# EUROPEAN PATENT SPECIFICATION

(11) **EP 4 337 277 B1**
(45) Date of publication and mention of the grant of the patent: **29.04.2026**
(21) Application number: 22725558.5
(22) Date of filing: 11.05.2022
(51) Int. Cl.: A61M 1/00

(54) **CONFORMABLE NEGATIVE-PRESSURE THERAPY DEVICE**
ANPASSBARE UNTERDRUCKTHERAPIEVORRICHTUNG
DISPOSITIF DE THÉRAPIE PAR PRESSION NÉGATIVE CONFORMABLE

(30) Priority: 13.05.2021 US 202163188197 P
(43) Date of publication of application: 20.03.2024
(73) Proprietor: KCI Manufacturing Unlimited Company, Athlone, Co. Westmeath, N37XF22 (IE)
(72) Inventor: PRATT, Benjamin Andrew, Bracknell Berkshire RG12 8HT (GB); SEDDON, James Killingworth, Bracknell Berkshire RG12 8HT (GB); LOCKE, Christopher Brian, Bracknell Berkshire RG12 8HT (GB)
(74) Representative: Simmons & Simmons
(86) International application number: PCT/IB2022/054374
(87) International publication number: WO 2022/238919

(56) References cited:
- US-A1- 2018 296 397
- US-A1- 2020 000 983

## Description

### TECHNICAL FIELD

The invention set forth in the appended claims relates generally to tissue treatment systems and more particularly, but without limitation, to a negative-pressure therapy device.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.
US2020/0000983 discloses a negative pressure wound treatment apparatus comprising a pump and attached canister.
US2018/0296397 discloses a negative pressure treatment apparatus with a pump integrated into a dressing.

### BRIEF SUMMARY

The invention is defined by the appended independent claim. A selection of optional features of the invention is set out in the dependent claims.

### BRIEF DESCRIPTION OF THE DRAWINGS

Figure 1 is a functional block diagram of an example embodiment of a therapy system that can provide negative-pressure treatment in accordance with this specification;
Figure 2 is a top isometric view of an example of a negative-pressure source that may be associated with some embodiments of the therapy system of Figure 1;
Figure 3 is a bottom isometric view of the example negative-pressure source of Figure 2;
Figure 4 is an exploded view of the negative-pressure source shown in Figure 2 and Figure 3 illustrating additional details that may be associated with some example embodiments;
Figure 5 is a cross-section view of a pump manifold that may be associated with some embodiments of the negative-pressure source shown in Figure 2 and Figure 3;
Figure 6 is an isometric back view of the negative-pressure source of Figure 2 illustrating additional details that may be associated with some example embodiments;
Figure 7 is a top isometric view of the negative-pressure source of Figure 2 illustrating additional details that may be associated with some example embodiments;
Figure 8 is a bottom isometric view of the negative-pressure source of Figure 2 illustrating additional details that may be associated with some example embodiments;
Figure 9 is a cross-section view of the negative-pressure source shown in Figure 7;
Figure 10 is an isometric view of another example of a negative-pressure source that may be associated with some example embodiments of the therapy system of Figure 1;
Figure 11 is a top view of the negative-pressure source of Figure 10;
Figure 12 is an exploded view of another example of a negative-pressure source that may be associated with some example embodiments of the therapy system of Figure 1;
Figure 13 is a cross-section view of the negative-pressure source of Figure 12 as assembled; Figure 14 is a front view of another example of a negative-pressure source that may be associated with some example embodiments of the therapy system of Figure 1;
Figure 15 is a cross-section view of the negative-pressure source of Figure 14;
Figure 16 is a cross-section view of another example of a negative-pressure source that may be associated with some example embodiments of the therapy system of Figure 1; and
Figure 17 is an exploded view of another example of a negative-pressure source that may be associated with some example embodiments of the therapy system of Figure 1.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure. or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

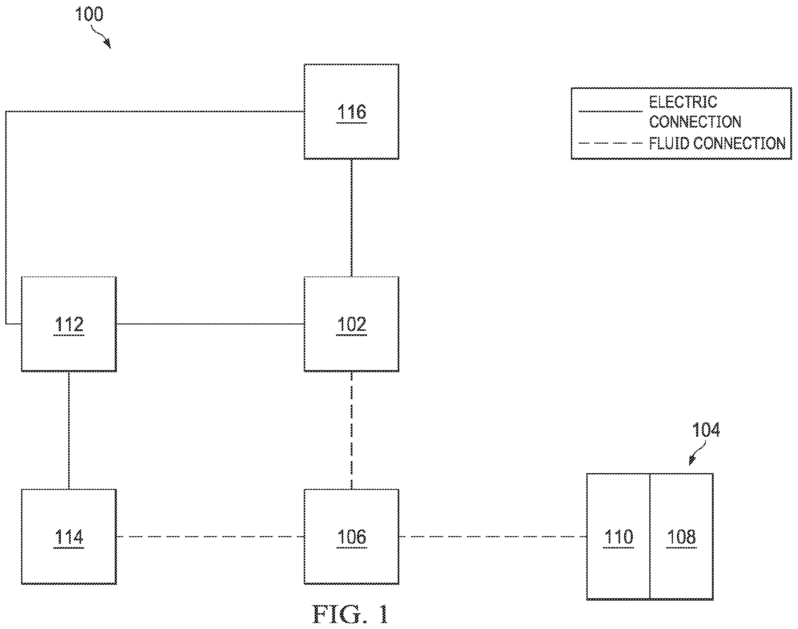

Figure 1 is a simplified functional block diagram of an example embodiment of a therapy system 100 that can provide negative-pressure therapy to a tissue site in accordance with this specification. The therapy system 100 may include a source or supply of negative pressure, such as a negative-pressure source 102, and one or more distribution components. A distribution component is preferably detachable and may be disposable, reusable, or recyclable. A dressing, such as a dressing 104, and a fluid container, such as a container 106, are examples of distribution components that may be associated with some examples of the therapy system 100. As illustrated in the example of Figure 1, the dressing 104 may comprise or consist essentially of a tissue interface 108, a cover 110, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 104. For example, such a dressing interface may be a SENSAT.R.A.C.^{™} Pad available from Kinetic Concepts, Inc. of San Antonio, Texas.

The therapy system 100 may also include a regulator or controller, such as a controller 112. Additionally, the therapy system 100 may include sensors to measure operating parameters and provide feedback signals to the controller 112 indicative of the operating parameters. As illustrated in Figure 1, for example, the therapy system 100 may include a first sensor 114 and a second sensor 116 coupled to the controller 112.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 102 may be combined with the controller 112 and other components into a therapy unit.

In general, components of the therapy system 100 may be coupled directly or indirectly. For example, the negative-pressure source 102 may be directly coupled to the container 106 and may be indirectly coupled to the dressing 104 through the container 106. Coupling may include fluid, mechanical, thermal, electrical, or chemical coupling (such as a chemical bond), or some combination of coupling in some contexts. For example, the negative-pressure source 102 may be electrically coupled to the controller 112 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 102, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 102 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 106 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 112, may be a microprocessor or computer programmed to operate one or more components of the therapy system 100, such as the negative-pressure source 102. In some embodiments, for example, the controller 112 may be a microcontroller, which generally comprises an integrated circuit containing a processor core and a memory programmed to directly or indirectly control one or more operating parameters of the therapy system 100. Operating parameters may include the power applied to the negative-pressure source 102, the pressure generated by the negative-pressure source 102, or the pressure distributed to the tissue interface 108, for example. The controller 112 is also preferably configured to receive one or more input signals, such as a feedback signal, and programmed to modify one or more operating parameters based on the input signals.

Sensors, such as the first sensor 114 and the second sensor 116, are generally known in the art as any apparatus operable to detect or measure a physical phenomenon or property, and generally provide a signal indicative of the phenomenon or property that is detected or measured. For example, the first sensor 114 and the second sensor 116 may be configured to measure one or more operating parameters of the therapy system 100. In some embodiments, the first sensor 114 may be a transducer configured to measure pressure in a pneumatic pathway and convert the measurement to a signal indicative of the pressure measured. In some embodiments, for example, the first sensor 114 may be a piezo-resistive strain gauge. The second sensor 116 may optionally measure operating parameters of the negative-pressure source 102, such as a voltage or current, in some embodiments. Preferably, the signals from the first sensor 114 and the second sensor 116 are suitable as an input signal to the controller 112, but some signal conditioning may be appropriate in some embodiments. For example, the signal may need to be filtered or amplified before it can be processed by the controller 112. Typically, the signal is an electrical signal, but may be represented in other forms, such as an optical signal.

The tissue interface 108 can be generally adapted to partially or fully contact a tissue site. The tissue interface 108 may take many forms, and may have many sizes, shapes, or thicknesses, depending on a variety of factors, such as the type of treatment being implemented or the nature and size of a tissue site. For example, the size and shape of the tissue interface 108 may be adapted to the contours of deep and irregular shaped tissue sites. Any or all of the surfaces of the tissue interface 108 may have an uneven, coarse, or jagged profile.

In some embodiments, the tissue interface 108 may comprise or consist essentially of a manifold. A manifold in this context may comprise or consist essentially of a means for collecting or distributing fluid across the tissue interface 108 under pressure. For example, a manifold may be adapted to receive negative pressure from a source and distribute negative pressure through multiple apertures across the tissue interface 108, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections: and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 108 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch (1 inch = 2.54 cm)) may be particularly suitable for some types of therapy. The tensile strength of the tissue interface 108 may also vary according to needs of a prescribed therapy. For example, the tensile strength of foam may be increased for instillation of topical treatment solutions. The 25% compression load deflection of the tissue interface 108 may be at least 0.35 pounds (1 pound = 0.45359237 kilograms) per square inch, and the 65% compression load deflection may be at least 0.43 pounds per square inch. In some embodiments, the tensile strength of the tissue interface 108 may be at least 10 pounds per square inch. The tissue interface 108 may have a tear strength of at least 2.5 pounds per inch. In some embodiments, the tissue interface 108 may be foam comprised of polyols such as polyester or polyether, isocyanate such as toluene diisocyanate, and polymerization modifiers such as amines and tin compounds. In some examples, the tissue interface 108 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 108 may also vary according to needs of a prescribed therapy. For example, the thickness of the tissue interface 108 may be decreased to reduce tension on peripheral tissue. The thickness of the tissue interface 108 can also affect the conformability of the tissue interface 108. In some embodiments, a thickness in a range of about 5 millimeters to 10 millimeters may be suitable.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 110 may provide a bacterial barrier and protection from physical trauma. The cover 110 may also be constructed from a material that can reduce evaporative losses and provide a fluid seal between two components or two environments, such as between a therapeutic environment and a local external environment. The cover 110 may comprise or consist of, for example, an elastomeric film or membrane that can provide a seal adequate to maintain a negative pressure at a tissue site for a given negative-pressure source. The cover 110 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 110 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 110 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inspire 2327 polyurethane films, commercially available from Expopack Advanced Coatings, Wrexham, United Kingdom. In some embodiments, the cover 110 may comprise INSPIRE 2301 having an MVTR (upright cup technique) of 2600 g/m²/24 hours and a thickness of about 30 microns.

An attachment device may be used to attach the cover 110 to an attachment surface, such as undamaged epidermis, a gasket, or another cover. The attachment device may take many forms. For example, an attachment device may be a medically-acceptable, pressure-sensitive adhesive configured to bond the cover 110 to epidermis around a tissue site. In some embodiments, for example, some or all of the cover 110 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

In operation, the tissue interface 108 may be placed within, over, on, or otherwise proximate to a tissue site. If the tissue site is a wound, for example, the tissue interface 108 may partially or completely fill the wound, or it may be placed over the wound. The cover 110 may be placed over the tissue interface 108 and sealed to an attachment surface near a tissue site. For example, the cover 110 may be sealed to undamaged epidermis peripheral to a tissue site. Thus, the dressing 104 can provide a sealed therapeutic environment proximate to a tissue site, substantially isolated from the external environment, and the negative-pressure source 102 can reduce pressure in the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and this descriptive convention should not be construed as a limiting convention.

Negative pressure applied across the tissue site through the tissue interface 108 in the sealed therapeutic environment can induce macro-strain and micro-strain in the tissue site. Negative pressure can also remove exudate and other fluid from a tissue site, which can be collected in container 106.

In some embodiments, the controller 112 may receive and process data from one or more sensors, such as the first sensor 114. The controller 112 may also control the operation of one or more components of the therapy system 100 to manage the pressure delivered to the tissue interface 108. In some embodiments, controller 112 may include an input for receiving a desired target pressure and may be programmed for processing data relating to the setting and inputting of the target pressure to be applied to the tissue interface 108. In some example embodiments, the target pressure may be a fixed pressure value set by an operator as the target negative pressure desired for therapy at a tissue site and then provided as input to the controller 112. The target pressure may vary from tissue site to tissue site based on the type of tissue forming a tissue site, the type of injury or wound (if any), the medical condition of the patient, and the preference of the attending physician. After selecting a desired target pressure, the controller 112 can operate the negative-pressure source 102 in one or more control modes based on the target pressure and may receive feedback from one or more sensors to maintain the target pressure at the tissue interface 108.

In some embodiments, the controller 112 may have a continuous pressure mode, in which the negative-pressure source 102 is operated to provide a constant target negative pressure for the duration of treatment or until manually deactivated. Additionally or alternatively, the controller may have an intermittent pressure mode. For example, the controller 112 can operate the negative-pressure source 102 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 102, which can form a square wave pattern between the target pressure and atmospheric pressure.

In some example embodiments, the increase in negative-pressure from ambient pressure to the target pressure may not be instantaneous. For example, the negative-pressure source 102 and the dressing 104 may have an initial rise time. The initial rise time may vary depending on the type of dressing and therapy equipment being used. For example, some therapy systems may increase negative pressure at a rate of about 20-30 mmHg/second, and other therapy systems may increase negative pressure at a rate of about 5-10 mmHg/second. If the therapy system 100 is operating in an intermittent mode, the repeating rise time may be a value substantially equal to the initial rise time.

In some example dynamic pressure control modes, the target pressure can vary with time. For example, the target pressure may vary in the form of a triangular waveform, varying between a negative pressure of 50 and 135 mmHg with a rise rate of negative pressure set at a rate of 25 mmHg/min. and a descent rate set at 25 mmHg/min. In other embodiments of the therapy system 100, the triangular waveform may vary between negative pressure of 25 and 135 mmHg with a rise rate of about 30 mmHg/min. and a descent rate set at about 30 mmHg/min.

In some embodiments, the controller 112 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 112, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

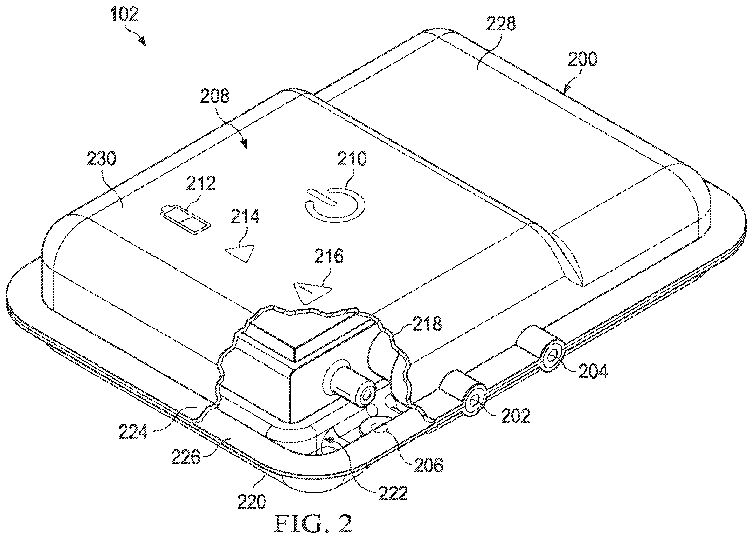

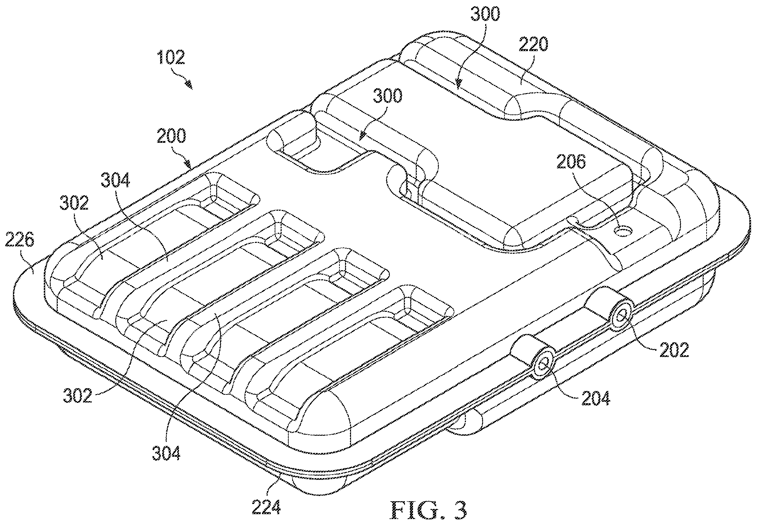

Figure 2 is a top isometric view of an example of a negative-pressure source 102 and Figure 3 is a bottom isometric view of the example negative-pressure source 102 of Figure 2. In some embodiments, the negative-pressure source 102 includes a case 200, a negative pressure port 202, an atmospheric pressure measurement port 204, and a pump exhaust port 206. As shown in Figure 2, the negative-pressure source 102 may include an interface 208 having one or more interface elements, such as for example, buttons or icons. As shown in the example of Figure 2, the interface 208 includes a power icon 210, a battery indicator icon 212, a start icon 214, and a warning icon 216.

The case 200 is formed from a first layer, such as a front layer 218, and a second layer, such as a rear layer 220. The front layer 218 and the rear layer 220 are coupled together to form the case 200 and an interior space 222 therein. The front layer 218 and the rear layer 220 are flexible and allow for the negative-pressure source 102 to be conformable. In some embodiments, the front layer 218 and the rear layer 220 are formed layers made from or including a polymer film. In the claimed embodiment, the formed front layer and the formed rear layer are made from a flexible polymer film. In some embodiments, the front layer 218 and the rear layer 220 may comprise a thermoplastic film or sheet. The front layer 218 and the rear layer 220 may comprise, for example, one or more of the following materials: thermoplastic polyurethane (TPU); polyethylene; polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. In some embodiments, one or more of the front layer 218 and the rear layer 220 may be fluid impermeable. For example, one or more of the front layer 218 and the rear layer 220 may be configured to prevent the passage of liquid and gas through the front layer 218 and/or the rear layer 220. In some embodiments, one or more of the front layer 218 and the rear layer 220 may be configured to prevent the passage of liquid, but allow the passage of gas or vapor, through the front layer 218 and/or the rear layer 220. In some embodiments, one or more of the front layer 218 and the rear layer 220 may be formed of a material that is liquid impermeable. In some embodiments, one or more of the front layer 218 and the rear layer 220 may be formed of a material that is gas impermeable. In some embodiments, one or more of the front layer 218 and the rear layer 220 may be formed of a material that is liquid impermeable but gas permeable.

In some embodiments, one or more of the front layer 218 and the rear layer 220 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties. In some example embodiments, one or more of the front layer 218 and the rear layer 220 may be a polymer sheet, such as a polyurethane film, that is permeable to water vapor but impermeable to liquid. A polyurethane film that allows water vapor to evaporate may allow any fluids that enter into the interior space 222 to evaporate out of the negative-pressure source 102.

In some embodiments, a flange 224 extends around the periphery of the front layer 218 and a flange 226 extends around the periphery of the rear layer 220. The front layer 218 and the rear layer 220 may be coupled together at their respective flanges 224, 226. As shown in Figure 2 and Figure 3, the negative pressure port 202 and the atmospheric pressure measurement port 204 are located between the flange 224 of the front layer 218 and the flange 226 of the rear layer 220.

Suitable bonds between the front layer 218 and the rear layer 220 may include pressure-sensitive adhesive (reactive and non-reactive types); hot melt adhesive (spray applied or deployed as a film, woven, or non-woven); hot press lamination; or flame lamination. In some embodiments, the front layer 218 and the rear layer 220 may be welded together. For example, the flange 224 of the front layer 218 and the flange 226 of the rear layer 220 may be welded together using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. In some embodiments, coextruded tie layers may be used, for example, where one layer is a polyethylene-compatible film and the other layer is a polyurethane-compatible film. In some additional or alternative embodiments, a polyethylene film may be surface treated, such as corona-treated or plasma-treated, to enhance its ability to bond to polar films. An ethyl methyl acrylate (EMA) film may also have suitable hydrophobic and welding properties for some configurations.

In some embodiments, the case 200 may be fluid impermeable. For example, the case 200 may be configured to prevent the passage of liquid and gas through the case 200. In some embodiments, the case 200 may be configured to prevent the passage of liquid, but allow the passage of gas or vapor, from inside the interior space through the case 200 to the environment outside the case 200. In some embodiments, the case 200 may be formed of a material that is liquid impermeable. In some embodiments, the case 200 may be formed of a material that is gas impermeable. In some embodiments, the case 200 may be formed of a material that is liquid impermeable but gas permeable.

The front layer 218 and the rear layer 220 may also be formed to have a desired shape and/or features. The front layer 218 and/or the rear layer 220 may be formed to have regions at different elevations, such as for example, positive features and negative features. Negative features formed into one side of the front layer 218 result in positive features extending from the opposite side of the front layer 218. Likewise, negative features formed into one side of the rear layer 220 result in positive features extending from the opposite side of the rear layer 220. For example, as shown in Figure 2, the front layer 218 may be formed to have a ledge area 228 and an interface area 230, where the ledge area 228 and the interface area 230 are at different elevations with respect to the flange 224. As another example, as shown in Figure 3, the rear layer 220 may be formed to have one or more channels 300, one or more depressions 302, and one or more ribs 304, where the channels 300, the depressions 302 and the ribs 304 may be at different elevations with respect to the flange 226. In some embodiments, the front layer 218 and/or the rear layer 220 may be formed with other features. In some embodiments, the front layer 218 and the rear layer 220 are formed using thermoforming operations, such as for example, vacuum forming or pressure forming.

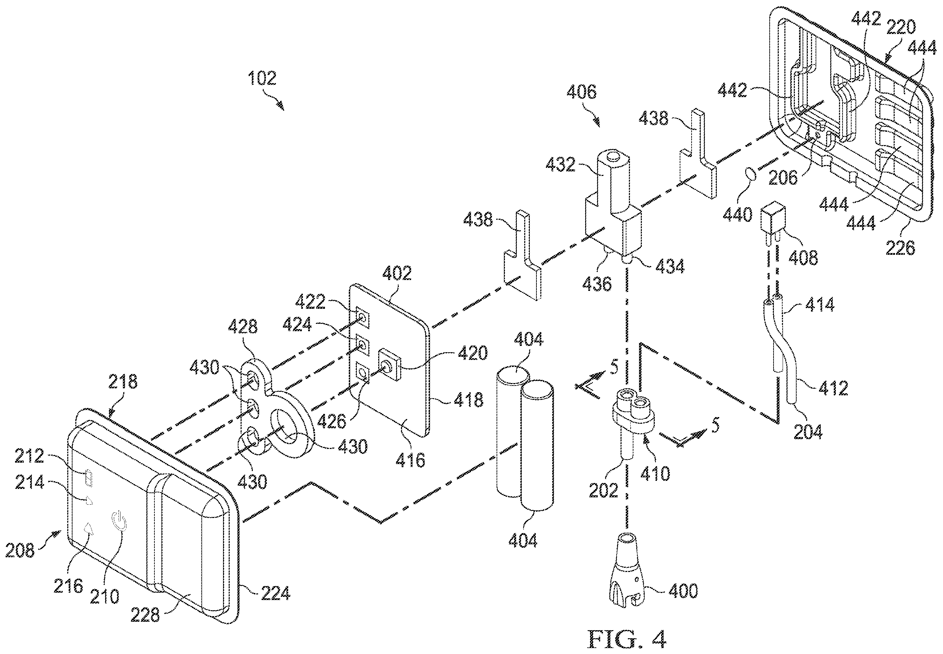

Figure 4 is an exploded view of the negative-pressure source 102 shown in Figure 2 and Figure 3 illustrating additional details that may be associated with some example embodiments. As shown in Figure 4, the interface 208 is located on the front layer 218. The exhaust port 206 of the negative-pressure source 102 is disposed in the rear layer 220. In some embodiments, the exhaust port 206 may be disposed in the front layer 218. In other embodiments, the exhaust port 206 may be located between the front layer 218 and the rear layer 220. In some embodiments, a connector, such as for example, a luer connector 400 maybe coupled to the negative pressure port 202.

The negative-pressure source 102 further includes a printed circuit board 402, a source of electrical power, such as a battery 404, a pump 406, and a pressure transducer 408. The negative-pressure source 102 may further include one or more fluid conductors, such as, for example, a pump manifold 410, an ambient pressure conduit 412, and a pump pressure conduit 414. The pump 406, printed circuit board 402, and the battery 404 are configured to be located between the front layer 218 and the rear layer 220 and within the interior space 222 formed by the front layer 218 and the rear layer 220.

The printed circuit board 402 has a first side 416 and a second side 418. The first side 416 of the printed circuit board 402 may face the front layer 218 and the second side 418 of the printed circuit board 402 may face the rear layer 220. On the first side 416 of the printed circuit board 402 may be a power button 420 and one or more light emitting diodes (LED), such as a battery indicator LED 422, a start LED 424, and a warning LED 426. The power button 420, the battery indicator LED 422, the start LED 424, and the warning LED 426 are configured to be located under the power icon 210, the battery indicator icon 212, the start icon 214, and the warning icon 216, respectively, when the negative-pressure source 102 is assembled. The power button 420 may include an LED.

In some embodiments, the pressure transducer 408 may be may be a surface-mount component on the second side 418 of the printed circuit board 402. The pump 406 and the battery 404 may be proximate to the second side 418 of the printed circuit board 402 between the printed circuit board 402 and the rear layer 220. The battery 404, pump 406, pressure transducer 408, power button 420, battery indicator LED 422, start LED 424, and warning LED 426 are electrically coupled to the printed circuit board 402.

The negative-pressure source 102 may further include a light mask and button bolster 428. The light mask and button bolster 428 is configured to be coupled to the first side 416 of the printed circuit board 402 and the front layer 218. The light mask and button bolster 428 has corresponding apertures 430 for each of the power button 420, the battery indicator LED 422, the start LED 424, and the warning LED 426. The light mask and button bolster 428 prevents light bleed between the LEDs. The light mask and button bolster 428 is configured to seal the LEDs and the power button 420 from the interior space 222. In some embodiments, the light mask and button bolster 428 may be formed of a compressible material, such as for example, a closed-cell foam. The light mask and button bolster 428 may be compressed when the power button 420 is pressed. In some embodiments, the light mask and button bolster 428 may be congruent with the printed circuit board 402. In some embodiments, one or more of the front layer 218 and the rear layer 220 are transparent. In some embodiments, one or more of the front layer 218 and the rear layer 220 are translucent. In some embodiments, one or more of the front layer 218 and the rear layer 220 are opaque. In some embodiments, one or more of the front layer 218 and the rear layer 220 may be clear, colored, or printed. For example, the rear layer 220 may be opaque and the front layer 218 may be translucent. The translucent front layer 218 may allow light from one or more of the power button 420, the battery indicator LED 422, the start LED 424, and the warning LED 426 to shine through the front layer 218. In another example, the front layer 218 and the rear layer 220 are transparent to allow for easy inspection of the interior space 222 and the components therein for possible fluid contamination.

In some embodiments, the pump 406 is a diaphragm pump. The pump 406 includes a pump body 432, a fluid inlet 434, and a fluid outlet 436. The pump 406 is electrically coupled with the printed circuit board 402 and the battery 404. The fluid inlet 434 is configured to be in fluid communication with the negative pressure port 202 and the fluid outlet 436 is configured to be in fluid communication with the pump exhaust port 206. The pump manifold 410 is configured to be coupled to the fluid inlet 434. The pump 406 may be coupled to the printed circuit board 402 by, for example, double-sided adhesive tape 438. In some embodiments, the pump 406 may be coupled to the rear layer 220 by, for example, double-sided adhesive tape 438. The double-sided tape 438 may have a shape similar to that of the sides of the pump body 432 facing the printed circuit board 402 and the rear layer 220.

The fluid outlet 436 and the exhaust port 206 are fluidly coupled to the interior space 222. The exhaust port 206 is configured to allow fluid from the fluid outlet 436 to be exhausted to the atmosphere. In some embodiments, the exhaust port 206 is sized such that it does not affect the performance of the pump 406 but causes a back pressure that inflates the interior space 222 between the front layer 218 and the rear layer 220. This may provide offloading when the negative-pressure source 102 is pressed against a patient. In some embodiments, the negative-pressure source 102 further includes a filter 440 configured to be disposed over the exhaust port 206. The filter 440 may be coupled to the side of the rear layer 220 facing the front layer 218. The filter 440 is configured to substantially preclude the passage of odors from the tissue site out of the negative-pressure source 102. The filter 440 may also be hydrophobic to substantially preclude the passage of liquids into and/or out of the negative-pressure source 102. The filter 440 is also configured to provide a desired level of backpressure to the pump 406. In some embodiments, the filter 440 may be comprised of a carbon material in the form of a layer or particulate. For example, the filter 440 may comprise a woven carbon cloth filter such as those manufactured by Chemviron Carbon, Ltd. of Lancashire, United Kingdom (www.chemvironcarbon.com). The filter 440 may be comprised of a material that is liquid impermeable and vapor permeable. For example, the primary hydrophobic filter 1005 may comprise a material manufactured under the designation MMT-314 by W.L. Gore & Associates, Inc. of Newark, Delaware, United States, or similar materials. The filter 440 may be provided in the form of a membrane or layer. In some embodiments, the back pressure imposed by the filter 440 is taken into account when sizing the exhaust port 206 to provide the desired back pressure to the pump 406 to cause inflation of the negative-pressure source 102 during operation of the pump 406.

With continued reference to Figure 4, the shapes and/or features formed into the front layer 218 and the rear layer 220 aid in locating and/or securing in place the internal components of the negative pressure source 102. In some embodiments, such features may be considered locating features. For example, the channels 300 (see FIG. 3) formed as a negative feature into the rear layer 220 form one or more walls 442. The walls 442 may be shaped to follow the outline of the pump body 432 and may form an area into which the pump body 432 may be located when the negative-pressure source 102 is assembled. Additionally, for example, the depressions 302 (see FIG. 3) formed as a negative feature into the rear layer 220 form protrusions 444. The protrusions 444 may be shaped to follow the shape of the batteries 404 and may form an area into which the batteries 404 may be located when the negative-pressure source 102 is assembled. Additionally, the ledge area 228 of the front layer 218 may also cooperate with the protrusions 444 to retain the batteries 404 in the desired location within the negative-pressure source 102.

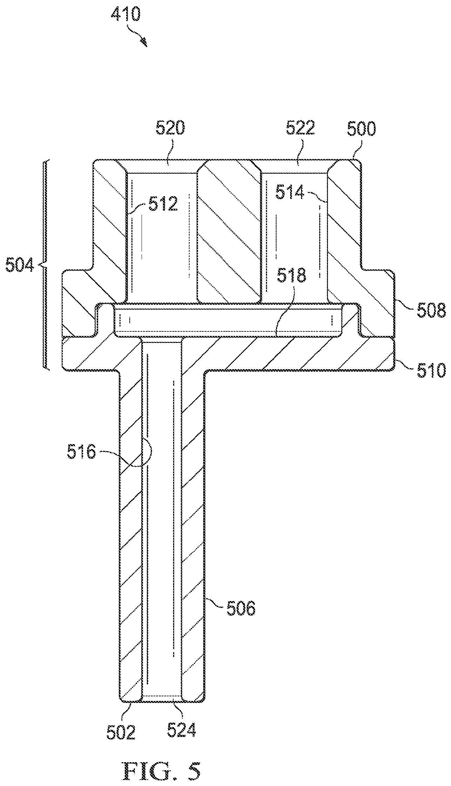

Now with reference to Figure 5, the pump manifold 410 of Figure 4 is shown in cross-section. The pump manifold 410 includes a first end 500 and a second end 502 opposite the first end 500. The pump manifold 410 includes a manifold body 504 and an inlet pipe 506 extending therefrom. The manifold body 504 is at the first end 500 and the inlet pipe 506 terminates at the second end 502. The manifold body 504 may include a first manifold body portion 508 and a second manifold body portion 510 configured to be coupled together. In some embodiments, the manifold body 504 may be formed of a single body. The first manifold body portion 508 includes a first passageway 512 and a second passageway 514. The second manifold body portion 510 includes a third passageway 516 extending through the inlet pipe 506. The second manifold body portion 510 also includes a bypass passageway 518. In some embodiments, the bypass passageway 518 is formed by the cooperation of a recessed portion of the second manifold body portion 510 and the first manifold body portion 508. The first passageway 512 and second passageway 514 are fluidly coupled by the bypass passageway 518. The first passageway 512 terminates in a first outlet port 520 at the first end 500 and the second passageway 514 terminates in a second outlet port 522 at the first end 500. The third passageway 516 terminates in an inlet port 524 at the second end 502. The first passageway 512, the second passageway 514, and the third passageway 516 are fluidly coupled to one another by the bypass passageway 518.

The manifold 410 is configured to fluidly couple the pump 406, the pressure transducer 408, and the negative-pressure port 202. For example, the fluid inlet 434 of the pump 406 is configured to be fluidly coupled with the first passageway 512. In some embodiments, at least a portion of the fluid inlet 434 is configured to be inserted through the first outlet port 520 and into the first passageway 512. The pump pressure conduit 414 is configured to be fluidly coupled with the second passageway 512. In some embodiments, at least a portion of the pump pressure conduit 414 is configured to be inserted through the second outlet port 522 and into the second passageway 514. The pump manifold 410 serves to supply negative pressure generated by the pump 406 to outside the negative-pressure source 102. The inlet port 524 of the manifold 410 serves as the negative-pressure port 202 of the negative-pressure source 102.

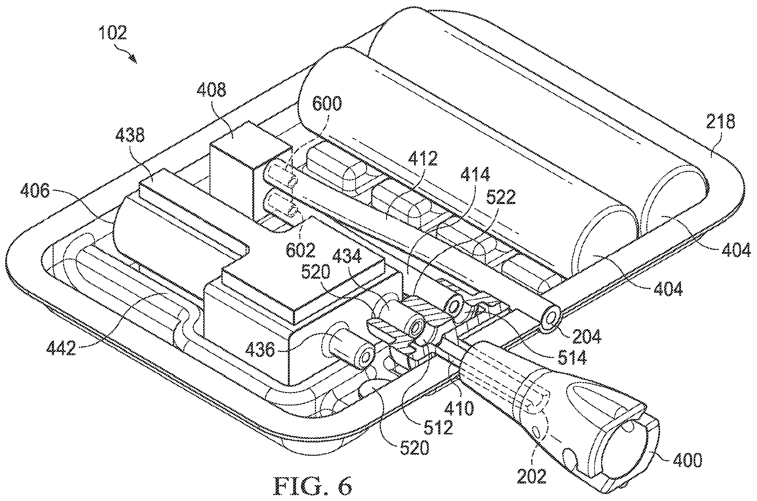

Figure 6 is an isometric back view of the negative-pressure source 102 of Figure 2 showing additional details of some embodiments. Several components, including the rear layer 220 and the printed circuit board 402, are not shown in Figure 6 for clarity purposes. As shown in Figure 6, the pressure transducer 408 includes a first port 600 and a second port 602. The first port 600 is configured to be coupled to the ambient environment outside the negative-pressure source 102 by a fluid path isolated from the interior space 222. The pressure transducer 408 is configured to measure the ambient pressure outside the negative-pressure source 102 via the first port 600. In some embodiments, the first port 600 is coupled to the ambient environment by the ambient pressure conduit 412. For example, the ambient pressure conduit 412 has a first end coupled with the first port 600 and a second end coupled to the ambient environment. The second end of the ambient pressure conduit 412 serves as the atmospheric pressure measurement port 204. In some embodiments, a filter may be disposed proximate to the second end of the ambient pressure conduit 412.

The second port 602 of the pressure transducer 408 is configured to be coupled to the fluid inlet 434. The pressure transducer 408 is configured to measure the negative pressure supplied by the pump 406 via the second port 602. In some embodiments, the second port 602 is coupled to the tissue site by the pump pressure conduit 414 and the pump manifold 410. For example, the pump pressure conduit 414 has a first end coupled with the second port 602 and a second end is inserted through the second outlet port 522 and into the second passageway 514 of the pump manifold 410.

As further shown in Figure 6, pump 406 is fluidly coupled with the pump manifold 410 and the pressure transducer 408. The fluid inlet 434 is inserted through the first outlet port 520 and into the first passageway 512 of the pump manifold 410. Negative pressure generated by the pump 406 may be delivered to a tissue site via the first passageway 512 and to the pressure transducer 408 via the first passageway 512, the bypass passageway 518, the second passageway 514, and the pump pressure conduit 414. The inlet port 524 of the pump manifold 410 serves as the negative pressure port 202.

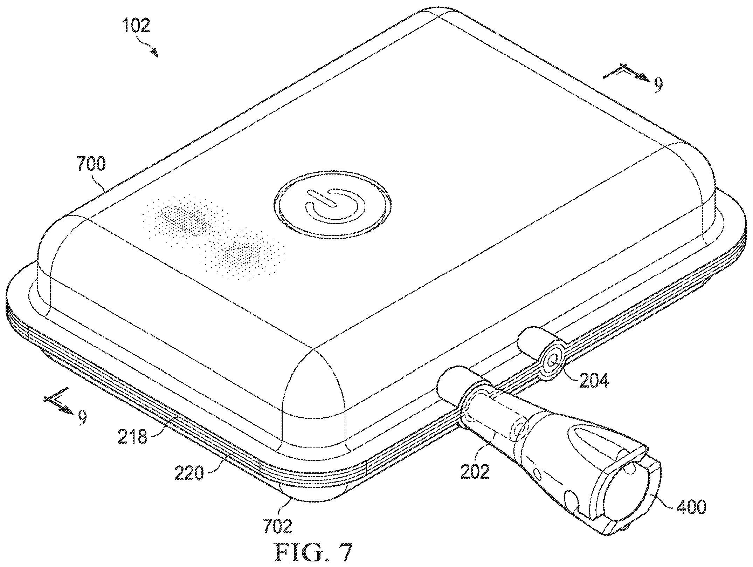

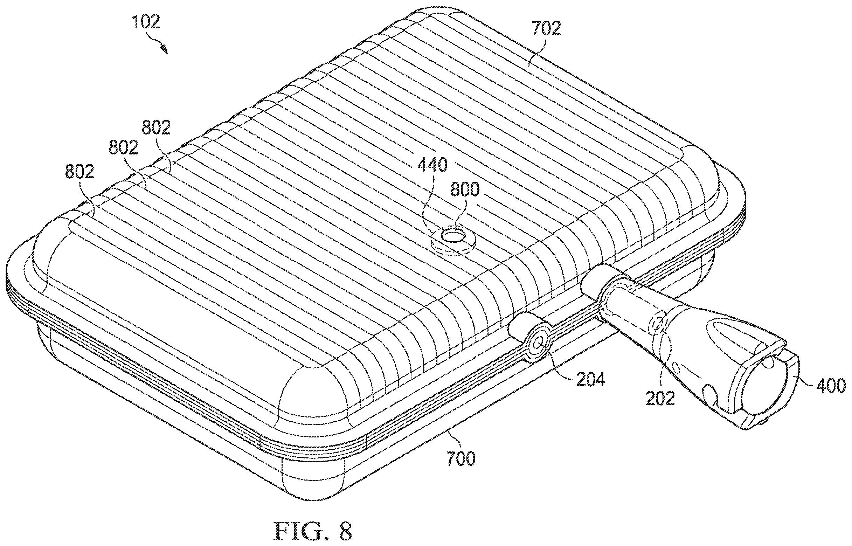

Figure 7 is a top isometric view and Figure 8 is a bottom isometric view of an embodiment of the example negative-pressure source 102 of Figure 2. As shown in Figure 7, the negative-pressure source 102 may further include a third layer, such as front outer layer 700, and a fourth layer, such as rear outer layer 702. The front outer layer 700 and the rear outer layer 702 may be coupled to the front layer 218 and the rear layer 220, respectively. In some embodiments, the front outer layer 700 and the rear outer layer 702 are formed from or include the same materials as the front layer 218 and the rear layer 220.

The front outer layer 700 and the rear outer layer 702 are flexible and allow for the negative-pressure source 102 to be conformable. In some embodiments, the front outer layer 700 and the rear outer layer 702 are formed layers made from or including a polymer film. In some embodiments, the front outer layer 700 and the rear outer layer 702 may comprise a thermoplastic film or sheet. The front outer layer 700 and the rear outer layer 702 may comprise, for example, one or more of the following materials: thermoplastic polyurethane (TPU); polyethylene; polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. In some embodiments, one or more of the front outer layer 700 and the rear outer layer 702 may be fluid impermeable. For example, one or more of the front outer layer 700 and the rear outer layer 702 may be configured to prevent the passage of liquid and gas through the front outer layer 70 and/or the rear outer layer 702. In some embodiments, one or more of the front outer layer 700 and the rear outer layer 702 may be configured to prevent the passage of liquid, but allow the passage of gas or vapor, through the front outer layer 700 and/or the rear outer layer 702. In some embodiments, one or more of the front outer layer 700 and the rear outer layer 702 may be formed of a material that is liquid impermeable. In some embodiments, one or more of the front outer layer 700 and the rear outer layer 702 may be formed of a material that is gas impermeable. In some embodiments, one or more of the front outer layer 700 and the rear outer layer 702 may be formed of a material that is liquid impermeable but gas permeable.

In some embodiments, one or more of the front outer layer 700 and the rear outer layer 702 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties. In some example embodiments, one or more of the front outer layer 700 and the rear outer layer 702 may be a polymer sheet, such as a polyurethane film, that is permeable to water vapor but impermeable to liquid. In some embodiments, the front layer 218, the rear layer 220, the front outer layer 700, and the rear outer layer 702 are formed of a material that is permeable to water vapor but impermeable to liquid. Polyurethane films that allow water vapor to evaporate may allow any fluids that enter into the interior space 222 to evaporate out of the negative-pressure source 102.

In some embodiments, one or more of the front outer layer 700 and the rear outer layer 702 are transparent. In some embodiments, one or more of the front outer layer 700 and the rear outer layer 702 are translucent. In some embodiments, one or more of the front outer layer 700 and the rear outer layer 702 are opaque. In some embodiments, one or more of front outer layer 700 and the rear outer layer 702 may be clear, colored, or printed. For example, the front outer layer 700 may be opaque and the rear outer layer 702 may be translucent. In another example, the front outer layer 700 and the rear outer layer 702 are transparent to allow for easy inspection of the inside of the negative-pressure source 102 and the components therein for possible fluid contamination.

The front outer layer 700 and the rear outer layer 702 may provide an outer shell for the negative-pressure source 102. The front outer layer 700 and the rear outer layer 702 may enhance the aesthetic appearance of the negative-pressure source 102. The front outer layer 700 and the rear outer layer 702 may make the negative-pressure source 102 appear similar to an injection molded product. The front outer layer 700 and the rear outer layer 702 may also improve robustness of the negative-pressure source, for example, by increasing puncture resistance. Additionally, the front outer layer 700 may provide additional diffusion of the LEDs of the interface 208. In some embodiments, the negative-pressure source 102 may include only a front outer layer 700 or a rear outer layer 702.

Figure 8 is a bottom isometric view of an embodiment of the example negative-pressure source 102 of Figure 7. As shown in Figure 8, the rear outer layer 702 includes an exhaust port 800 that is fluidly coupled with the exhaust port 206 in the second layer 220. In some embodiments, the negative-pressure source 102 may include a filter 440 fluidly coupled with the exhaust port 800. The filter 440 may be coupled to the inside of the rear outer layer 702 facing the rear layer 220. In some embodiments, the rear outer layer 702 may be formed with features, such as for example, one or more ribs extending across the surface of the rear outer layer 702. In some embodiments, the features may include a design or pattern.

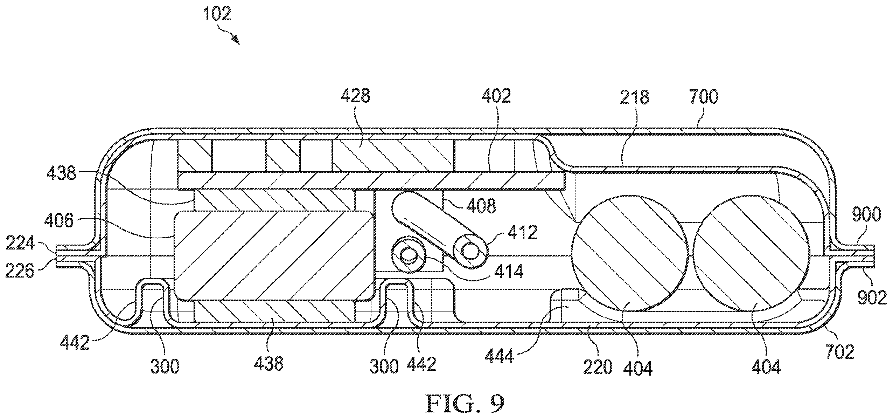

Figure 9 is a cross-section view of the negative-pressure source 102 shown in Figure 7. In some embodiments, a flange 900 extends around the periphery of the front outer layer 700 and a flange 902 extends around the periphery of the rear outer layer 702. The flanges 224, 900 of the front layer 218 and the front outer layer 700 may be coupled together and the flanges 226, 902 of the rear layer 220 and the rear outer layer 702 may be coupled together. Suitable bonds between the front layer 218, the rear layer 220, the front outer layer 700, and the rear outer layer 702 may include pressure-sensitive adhesive (reactive and non-reactive types); hot melt adhesive (spray applied or deployed as a film, woven, or non-woven); hot press lamination; or flame lamination. In some embodiments, the front layer 218, the rear layer 220, the front outer layer 700, and the rear outer layer 702 may be welded together. For example, the flanges 224, 226, 900, 902 may be welded together using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. In some embodiments, coextruded tie layers may be used, for example, where one layer is a polyethylene-compatible film and the other layer is a polyurethane-compatible film. In some additional or alternative embodiments, a polyethylene film may be surface treated, such as corona-treated or plasma-treated, to enhance its ability to bond to polar films. An ethyl methyl acrylate (EMA) film may also have suitable hydrophobic and welding properties for some configurations. In some embodiments, the flanges 224, 226, 900, 902 may be coupled together in a single operation, such as for example, by a four layer RF weld. In some embodiments, the flanges 224, 226 of the front layer 218 and the rear layer 220 may be coupled together in a first operation and then the flanges 900, 902 of the front outer layer 700 and the rear outer layer 702 may be coupled to the flanges 224, 226 of the front layer 218 and the rear layer 220, respectively, in a second operation.

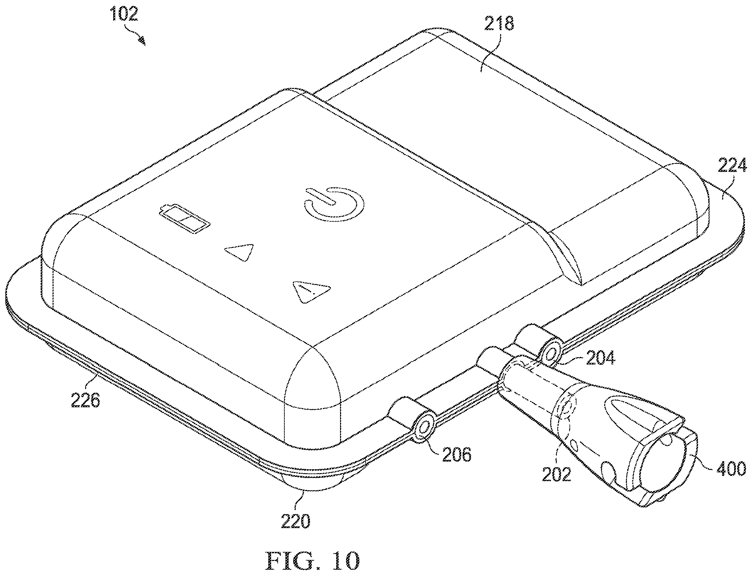

Figure 10 is an isometric view of another example of the negative-pressure source 102 that may be associated with some example embodiments of the therapy system 100. As shown in Figure 10, the negative-pressure port 202, the atmospheric pressure measurement port 204, and the exhaust port 206 are sandwiched between and coupled to the flanges 224, 226 of the front layer 218 and the rear layer 220. Sandwiching the negative-pressure port 202, the atmospheric pressure measurement port 204, and the exhaust port 206 between the flanges 224, 226 forms a secure pneumatic connection.

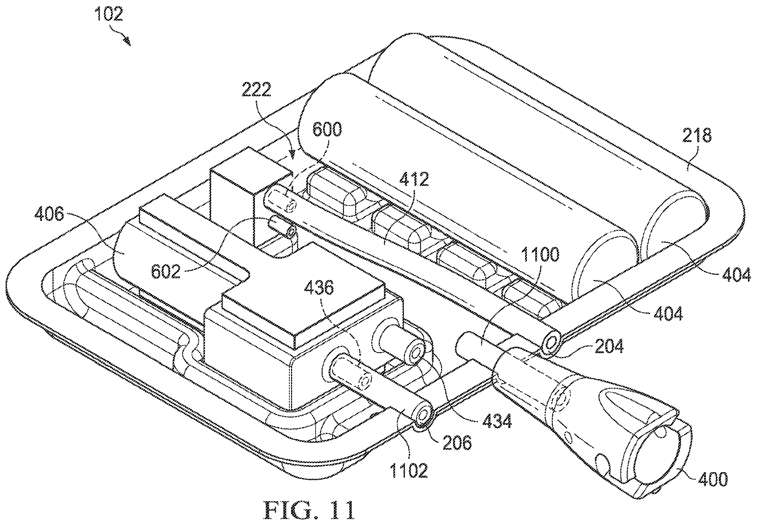

Figure 11 is a top view of the negative-pressure source 102 of Figure 10. Several components, including the rear layer 220 and the printed circuit board 402, are not shown in Figure 11 for clarity purposes. The negative-pressure source 102 includes the ambient pressure conduit 412, a negative-pressure conduit 1100, and a pump exhaust conduit 1102. The negative-pressure conduit 1100 is configured to fluidly couple the pump 406 with the tissue site to deliver negative pressure to the tissue site. The negative-pressure conduit 1100 has a first end disposed within the interior space 222 of the case 200 and a second end coupled to the ambient environment. In some embodiments, the second end of the negative-pressure conduit 1100 extends outward from the case 200 a distance. As shown in Figure 11, in some embodiments, the first end of the negative-pressure conduit 1100 and the fluid inlet 434 are fluidly coupled but a gap is provided between the first end of the negative-pressure conduit 1100 and the fluid inlet 434. As a result of the gap between the first end of the negative-pressure conduit 1100 and the fluid inlet 434, negative pressure generated by the pump 406 is applied to the interior space 222 of the case 200. In some embodiments, the negative-pressure inside the interior space 222 may be equal to the negative-pressure applied to the tissue site. The second port 602 of the pressure transducer 408 is fluidly coupled with the fluid inlet 434 via the interior space 222. Because the interior space 222 may be at the negative-pressure generated by the pump 406, the pressure transducer 408 can measure the negative-pressure generated by the pump 406.

The pump exhaust conduit 1102 is coupled to the fluid outlet 436 and is configured to exhaust fluid from the pump 406 to the atmosphere outside of the negative-pressure source 102. For example, a first end of the pump exhaust conduit 1102 is coupled to the fluid outlet 436 and a second end of the pump exhaust conduit 1102 is coupled to the ambient environment. The second end of the pump exhaust conduit 1102 serves as the pump exhaust port 206.

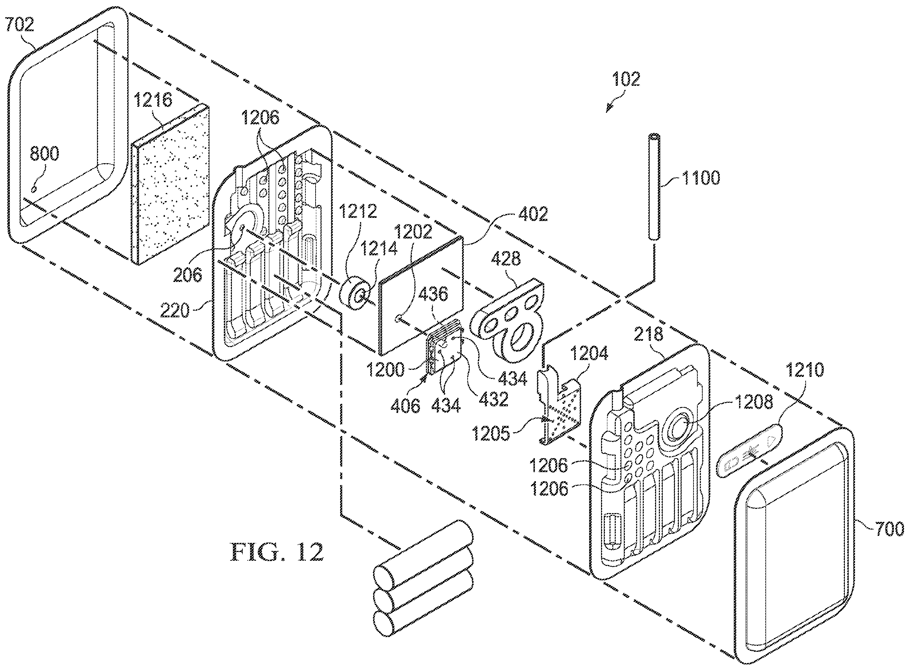

Figure 12 is an exploded view of another example of the negative-pressure source 102 that may be associated with some example embodiments of the therapy system 100. As shown in Figure 12, in the example negative-pressure source 102 the pump 406 is a piezoelectric pump. In some embodiments the pump 406 may be a Murata Microblower MZB3004T04 available from Murata Manufacturing Co., Ltd. of Kyoto, Japan. The pump 406 may have a generally square shaped pump body 432 with a flat face 1200 facing away from the printed circuit board 402. The pump 406 may have one or more fluid inlets 434 in the flat face 1200. The fluid outlet 436 of the pump 406 extends from the pump body 432 opposite the flat face 1200. The pump 406 may be mounted to the first side 416 of the printed circuit board 402. The printed circuit board 402 includes an aperture 1202 through which the fluid outlet 436 extends when the pump 406 is mounted on the printed circuit board 402. The negative-pressure source 102 further includes a pump cover 1204. The pump cover 1204 is configured to be coupled to the pump 406 and covers the flat face 1200. The pump cover 1204 is configured to couple the negative-pressure conduit 1100 to the pump 406. The pump cover 1204 may include a plurality of through holes 1204. The through holes 1205 are configured to allow air to flow through the pump cover 1204. The air flow through the holes 1205 may serve to cool the pump 406.

As further shown in Figure 12, in some embodiments, the front layer 218 and the rear layer 220 include a plurality of features formed therein such as, for example, projections, standoffs, open cells, or closed cells, which may provide support to the front layer 218 and the rear layer 220 to reduce or prevent collapse of the front layer 218 and the rear layer 220 during operation of the negative-pressure source 102. In response to the pump 406 operating, the interior space 222 between the front layer 218 and the rear layer 220 will be subjected to negative pressure, such as the therapy pressure. This negative pressure will tend to pull the front layer 218 and the rear layer 220 together, toward the printed circuit board 402. The features formed in the front layer 218 and the rear layer 220 can resist collapse of the front layer 218 and the rear layer 220. For example, the front layer 218 and the rear layer 220 include a plurality of projections 1206 extending from the front layer 218 and the rear layer 220 toward the printed circuit board 402. The projections 1206 may be thermoformed into the front layer 218 and the rear layer 220. Under negative pressure, the projections 1206 on the front layer 218 may contact the pump cover 1204 and the projections 1206 on the rear layer 220 may contact the printed circuit board 402. The projections 1206 have a stiffness sufficient to prevent complete collapse of the front layer 218 onto the pump cover 1204 and the rear layer 220 onto the printed circuit board 402, respectively. The projections 1206 are configured to prevent the front layer 218 from sealing the holes 1205 on the pump cover 1204. As shown in Figure 12, in some embodiments, the projections 1206 may be cylindrical in shape; however, other shaped projections may be utilized. In some embodiments, the plurality of features may be closed cells and may function similar to bubble wrap. The front layer 218 may further include a button cover or cap 1208 formed therein. The button cover 1208 may be aligned with the power button 420 on the printed circuit board. The button cover 1208 may be depressed to actuate the power button 420 and may spring back away from the power button 420 after being depressed.

As further shown in Figure 12, the negative-pressure source 102 further includes an icon mask 1210 that includes apertures forming icons through which light from the LED's (not shown in Figure 12) can shine. The icon mask 1210 may be located between the front layer 218 and the front outer layer 700. In some embodiments, the icon mask 1210 is coupled to the front outer layer 700. The front layer 218 may be transparent to allow light from the LED's (not shown in Figure 12) to shine through the front layer 218. The front outer layer 700 may be translucent such that the icons are not visible unless and until the LEDs are lit. In some embodiments, the power button 210 may be printed onto or embossed into the front outer layer 700.

In some embodiments, the negative-pressure source 102 further includes a sealing member, such as a gasket 1212, between the printed circuit board 402 and the rear layer 220. The gasket 1212 may have a ring shape with an aperture 1214. The gasket 1212 may be coupled to the printed circuit board 402 and the rear layer 220 and fluidly isolates the fluid outlet 436 and the exhaust port 206 from the interior space 222. The aperture 1214 fluidly couples the fluid outlet 436 with the exhaust port 206.

The negative-pressure source 102 may further include, in some embodiments, a layer 1216. The layer 1216 may be a manifold layer having a plurality of pathways, which can be interconnected to improve distribution or collection of fluids. In some illustrative embodiments, the manifold layer may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, the manifold layer may additionally or alternatively comprise projections that form interconnected fluid pathways. For example, the manifold layer may be molded to provide surface projections that define interconnected fluid pathways.

In some embodiments, the manifold layer may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch). The 25% compression load deflection of the tissue interface 108 may be at least 0.35 pounds per square inch, and the 65% compression load deflection may be at least 0.43 pounds per square inch. In some embodiments, the tensile strength of the manifold layer may be at least 10 pounds per square inch. The manifold layer may have a tear strength of at least 2.5 pounds per inch. In some embodiments, the manifold layer may be foam comprised of polyols such as polyester or polyether, isocyanate such as toluene diisocyanate. and polymerization modifiers such as amines and tin compounds. In some examples, the manifold layer may be reticulated polyurethane foam such as found in GRANUFOAM^{™} dressing or V.A.C. VERAFLO^{™} dressing, both available from Kinetic Concepts, Inc. of San Antonio, Texas. In some examples, the manifold layer may be a non-woven fabric such as Dry Web TDL2, available from Libeltex. The manifold layer may have a thickness in a range of about 1 millimeter to about 10 millimeters.

The layer 1216 may provide sound attenuation for the negative-pressure source 102, reducing the noise from the fluid outlet 436 of the pump 406. In some embodiments, the material properties and/or the thickness of the layer 1216 may be selected to filter out one or more frequencies emitted by operation of the pump 406. For example, where the layer 1216 is an open-cell reticulated polyurethane foam, one or more of the density, porosity, and the thickness of the layer 1216 may be selected to reduce the sound pressure level of one or more frequencies of the pump 406 during operation of the pump 406.

In some embodiments, the layer 1216 may be or include an absorbent configured to reduce the humidity of the exhaust flow from the fluid outlet 436. In some embodiments, the layer 126 includes both the manifold layer and the absorbent. The absorbent may prevent the formation of condensation within the negative-pressure source 102. In some embodiments, the absorbent may be coated on the rear layer 220 on the side of the rear layer 220 facing the rear outer layer 702. The absorbent may be any substance capable of storing a liquid, such as exudate. For example, the absorbent may form a chemical bond with exudate from the tissue site. Non-limiting examples of the absorbent include super absorbent fiber/particulates, hydrofibre, sodium carboxymethyl cellulose, and/or alginates. In some exemplary embodiments, the absorbent may be formed of a superabsorbent polymer (SAP). Generally, relative to their mass, SAPs can absorb and retain large quantities of liquid, and in particular water. SAPs may be used to hold and stabilize or solidify wound fluids. The SAPs used to form the absorbent may be of the type often referred to as "hydrogels," "superabsorbents," or "hydrocolloids." The SAPs may be formed into fibers or spheres to manifold reduced pressure until the SAPs become saturated. In some embodiments, the absorbent may comprise a substrate in which the SAPs may be dispersed as pellets throughout and/or embedded as a sheet-like layer.

The SAPs may be formed in several ways, for example, by gel polymerization, solution polymerization, or suspension polymerization. Gel polymerization may involve blending of acrylic acid, water, cross-linking agents, and ultraviolet (UV) initiator chemicals. The blended mixture may be placed into a reactor where the mixture is exposed to UV light to cause crosslinking reactions that form the SAP. The mixture may be dried and shredded before subsequent packaging and/or distribution. Solution polymerization may involve a water based monomer solution that produces a mass of reactant polymerized gel. The monomer solution may undergo an exothermic reaction that drives the crosslinking of the monomers. Following the crosslinking process, the reactant polymer gel may be chopped, dried, and ground to its final granule size. Suspension polymerization may involve a water-based reactant suspended in a hydrocarbon-based solvent. However, the suspension polymerization process must be tightly controlled and is not often used.

SAPs absorb liquids by bonding with water molecules through hydrogen bonding. Hydrogen bonding involves the interaction of a polar hydrogen atom with an electronegative atom. As a result, SAPs absorb water based on the ability of the hydrogen atoms in each water molecule to bond with the hydrophilic polymers of the SAP having electronegative ionic components. High absorbing SAPs are formed from ionic crosslinked hydrophilic polymers such as acrylics and acrylamides in the form of salts or free acids. Because the SAPs are ionic, they are affected by the soluble ionic components within the solution being absorbed and will, for example, absorb less saline than pure water. The lower absorption rate of saline is caused by the sodium and chloride ions blocking some of the water absorbing sites on the SAPs. If the fluid being absorbed by the SAP is a solution containing dissolved mineral ions, fewer hydrogen atoms of the water molecules in the solution may be free to bond with the SAP. Thus, the ability of an SAP to absorb and retain a fluid may be dependent upon the ionic concentration of the fluid being absorbed. For example, an SAP may absorb and retain de-ionized water up to 500 times the weight of the dry SAP. In volumetric terms, an SAP may absorb fluid volumes as high as 30 to 60 times the dry volume of the SAP. Other fluids having a higher ionic concentration may be absorbed at lower quantities. For example, an SAP may only absorb and retain a solution that is 0.9% salt (NaCl) up to 50 times the weight of the dry SAP. Since wound fluids contain salts, such as sodium, potassium, and calcium, the absorption capacity of the SAP may be reduced if compared to the absorption capacity of deionized water.

In some embodiments, the absorbent may comprise a KERRAMAX CARE^{™} SuperAbsorbent Dressing material available from Kinetic Concepts, Inc. of San Antonio, Texas. For example, the absorbent may comprise a superabsorbent laminate comprised of 304 g.s.m. FAVOR-PAC^{™} 230 superabsorbent powder glued by PAFRA^{™} 8667 adhesive between two layers of 50 g.s.m. LIDRO^{™} non-woven material. In some embodiments, the absorbent may comprise an absorbent available from Gelok International. In some embodiments, if the rear outer layer 702 has a high MVTR as described above, some of the fluids captured by the absorbent may evaporate and exit the negative-pressure source 102 through rear outer layer 702 as water vapor.

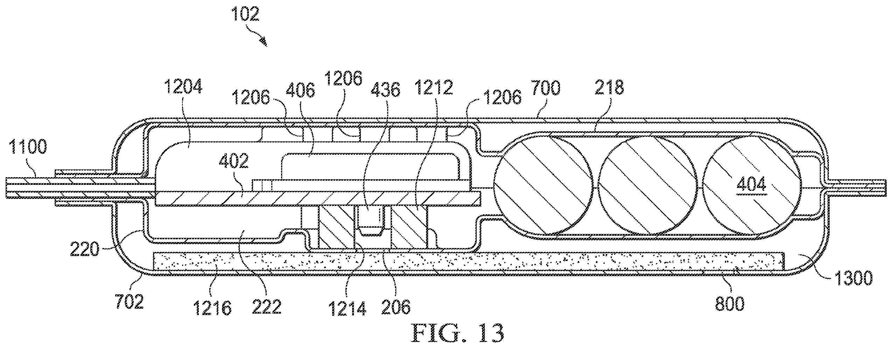

Figure 13 is a cross-section view of the negative-pressure source 102 of Figure 12 as assembled. As shown in Figure 13, the fluid outlet 436 of the pump 406 is fluidly isolated from the interior space 222 by the gasket 1212 and fluidly coupled with a second interior space 1300 formed by the rear layer 220 and the outer rear layer 702 via the aperture 1214 in the gasket 1212 and the pump exhaust port 206. The layer 1216 is disposed in the second interior space 1300 between the rear layer 220 and the outer rear layer 702. The fluid exhausted from the fluid outlet 436 of the pump 406 may flow through the pump exhaust port 206, the second interior space 1300, through the layer 1216, and out of the negative-pressure source 102 through the exhaust port 800. The projections 1206 are shown in contact with the pump cover 1204.

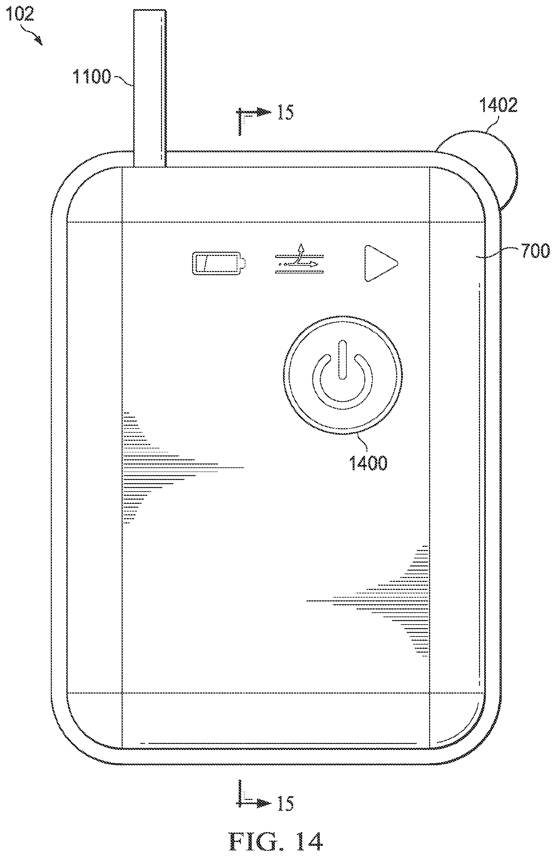

Figure 14 is a front view of another example of the negative-pressure source 102 that may be associated with some example embodiments of the therapy system 100. The front outer layer 700 includes an embossed power button 1400 that may aid in the user locating the power button 420. In some embodiments, one or more of the front outer layer 700, the rear outer layer 702, the front layer 218, and the rear layer 220 may be configured to be easily removed from the negative-pressure source 102 without damage to the remaining portions of the negative-pressure source 102. For example, as shown in Figure 14, in some embodiments, the front outer layer 700 includes a tab 1402 that may be pulled on to easily remove the front outer layer 700. For example, flanges 224, 226, 902 of the front layer 218, rear layer 220, and the rear outer layer 702 may be coupled by a three-layer RF weld, and the flange 900 of the front outer layer 700 may be coupled to the flange 224 of the front layer 218 by a separable adhesive bond. Removing the front outer layer 700 may allow removal of portions of the negative-pressure source 102 for disposal, recycling, and/or reuse. In some embodiments, for example, one or more of the batteries 404, the printed circuit board 402, the light mask and button bolster 428 could be removed at their end of life prior and may be replaced if the negative-pressure source 102 is going to be reused. For example, if the batteries 404 die, the front outer layer 700 may be removed and fresh batteries may be placed in the negative-pressure source 102 to allow continued use of the negative-pressure source 102. After the batteries 404 are replaced, the front outer layer 700, or a new front outer layer 700, may be coupled to the negative-pressure source 102 to seal the batteries 404 therein. The negative-pressure source 102 can then be used again.

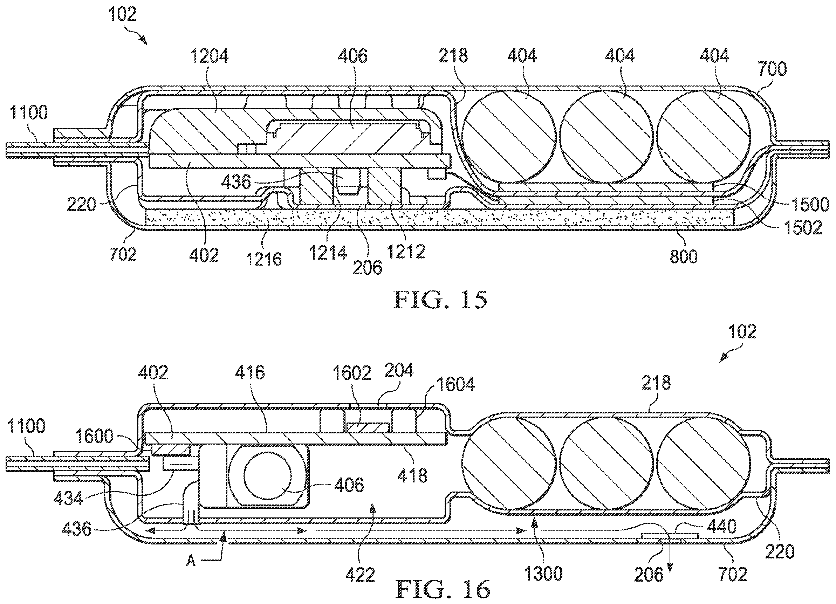

Figure 15 is a cross-section view of the negative-pressure source 102 of Figure 14. As shown in Figure 15, the batteries 404 are located between the front layer 218 and the front outer layer 700, wherein the front outer layer 700 is an outer layer. In some embodiments the electrical connection between the batteries 404 and the printed circuit board 402 may be wireless or noncontact. For example, the batteries 404 may be electrically coupled to a first inductive coil 1500 and the printed circuit board 402 may be electrically coupled to a second inductive coil 1502. The first coil 1500 and the second coil 1502 may be physically separated by a layer, such as the front layer 218. Power from the batteries 404 may be transmitted by induction from the first coil 1500 to the second coil 1502 to provide power to the printed circuit board 402 and the electrical components electrically coupled thereto. Using the inductive first coil 1500 and the second coil 1502 allows the batteries 404 to be sealed off from the printed circuit board 402. In some embodiments, the batteries 404 may be recharged via the first coil 1500 and/or the second coil 1502, extending the useful life of the negative-pressure source 102.

Figure 16 is a cross-section view of another example of the negative-pressure source 102 that may be associated with some example embodiments of the therapy system 100. As shown in Figure 16, the negative-pressure source 102 includes an absolute pressure sensor 1600 fluidly coupled with the interior space 222. The absolute pressure sensor 1600 is electrically coupled to the printed circuit board 402 and may be surface mounted to the second side 418 of the printed circuit board 402. Optionally, a relative pressure sensor 1602 may be electrically coupled to the printed circuit board 402. The relative pressure sensor 1602 may be surface mounted to the first side 416 of the printed circuit board 402. The relative pressure sensor 1602 may be fluidly coupled with the ambient environment outside of the negative-pressure source 102 via the atmospheric pressure measurement port 204. The atmospheric pressure measurement port 204 may be located in the front layer 218. A sealing member, such as a gasket 1604, may be included to fluidly isolate the relative pressure sensor 1602 from the interior space 222 and negative pressure therein. For example, the gasket 1604 may be located between the printed circuit board 402 and the front layer 218. The relative pressure sensor 1602 can allow for the measurement of relative negative pressure rather than just absolute pressure. The relative pressure sensor 1602 can allow for the desired application of negative pressure to a tissue site even if there are changes in altitude of the patient.

As further shown in Figure 16, the fluid outlet 436 of the pump 406 is fluidly isolated from the interior space 222 and fluidly coupled with the second interior space 1300 formed by the rear layer 220 and the outer rear layer 702. The fluid exhausted from the fluid outlet 436 of the pump 406 may flow through the second interior space 1300, through the filter 440, and out of the negative-pressure source 102 through the pump exhaust port 206 (as shown by arrows A). The exhaust port 206 in the outer rear layer 702 may be sized to impose a back pressure on the pump 406 that is configured to cause inflation of the second interior space 1606 during operation of the pump 406. Inflation of the second interior space 1300 may provide offloading of the negative-pressure source 102, which may increase patient comfort.

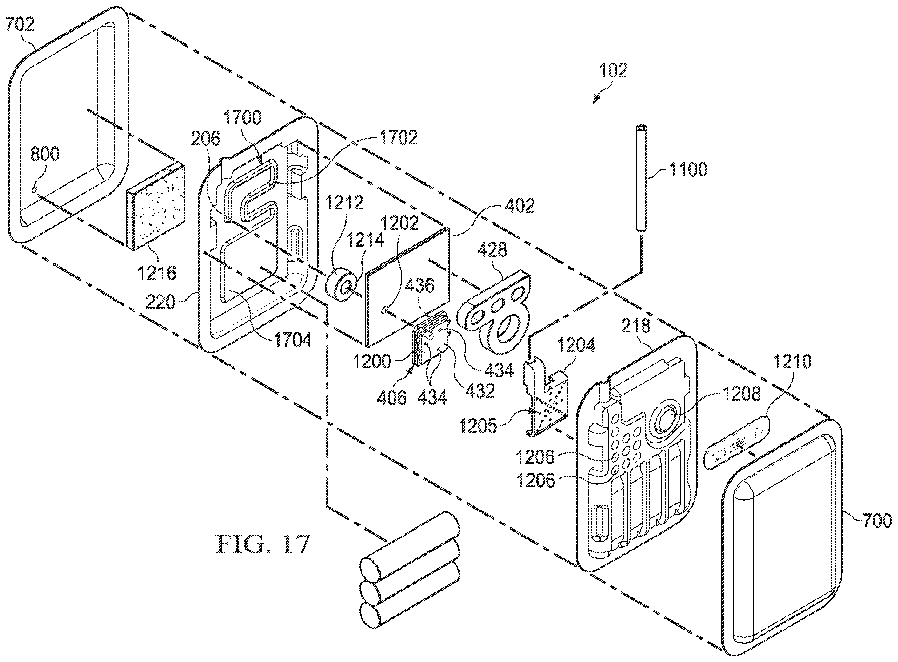

Figure 17 is an exploded view of another example of the negative-pressure source 102 that may be associated with some example embodiments of the therapy system 100. In some embodiments, the negative-pressure source 102 may include one or more acoustic tuning elements configured to alter and/or reduce the sound pressure level output of the negative-pressure source 102 during operation. In some embodiments, the one or more acoustic tuning elements may alter the frequency of sound exiting the exhaust port 800. For example, in some embodiments, the acoustic tuning elements may include a flow path 1700 having a flow channel 1702 and an expansion chamber 1704. The flow path 1700 is fluidly coupled with the pump exhaust port 206 in the rear layer 220 and the exhaust port 800 in the rear outer layer 702. The flow channel 1702 may have a tortuous or serpentine shape. The flow path 1700 is fluidly coupled with the pump outlet 436 and the ambient environment and is configured to reduce the noise made by the pump 406 and air exhausted by the pump 406. In some embodiments, the flow path 1700 may compress the air, changing the frequency of the sound emitted by the negative-pressure source 102. In some embodiments, the flow path 1700 may cause expansion of the air, changing the frequency of the sound emitted by the negative-pressure source 102.

The flow channel 1702 and the expansion chamber 1704 may be formed into the rear layer 220. As shown in Figure 17, the layer 1216 may be disposed in the expansion chamber 1704 and may provide sound attenuation for the negative-pressure source 102, reducing the noise from the fluid outlet 436 of the pump 406. In some embodiments, the material properties and/or the thickness of the layer 1216 may be selected to filter out one or more frequencies emitted by operation of the pump 406. For example, where the layer 1216 is an open-cell reticulated polyurethane foam, one or more of the density, porosity, and the thickness of the layer 1216 may be selected to reduce the sound pressure level of one or more frequencies of the pump 406 during operation of the pump 406. In some embodiments, the flow path 1700 may be formed by one or more baffles formed into the rear layer 220. The baffles may form a tortuous flow path between the exhaust port 800 and the fluid outlet 436 of the pump 406, the baffles configured to compress fluid flowing through the tortuous flow path and change a frequency of the fluid flow.

In some embodiments, inflation of at least a part of the negative-pressure source 102 under positive pressure may be achieved by including a sealed inflation chamber disposed in the negative-pressure source 102. The negative-pressure source 102 may also include a diverter valve fluidly coupled to the fluid outlet 436 of the pump 406, the inflation chamber, and the exhaust port 206/800. The diverter valve may be configured to selectively divert the fluid outlet between the exhaust port 206/800 and the inflation chamber. For example, the inflation chamber may be inflated by the pump 406 to a set pump stall pressure (e.g., 200 mmHg). Then the inflation chamber may be sealed by switching the diverter valve to venting the exhaust fluid from the pump 406 to the ambient environment. The inflation chamber may provide additional offloading of the negative-pressure source 102 if the negative-pressure source 102 is placed on or near a patient. If the negative-pressure source 102 includes a device or component that incorporates a high static magnetic flux, the spacing of the negative-pressure source 102 away from the patient may be increased if required by inflation of the inflation chamber.

The systems, apparatuses, and methods described herein may provide significant advantages. For example, constructing a negative-pressure source 102 using flexible film layers **,** as in the claimed embodiment, may eliminate the need for most injection molded parts. This results in a conformable negative-pressure source that may be easier to use, may provide offloading from the patient, may increase patient comfort, may be integrated into dressings, and with which disposal can be achieved in a socially and environmentally responsible manner. The negative-pressure source 102 described herein may also have simple device construction and may allow for easy rebranding and/or differentiation through thermoforming and/or printing designs and logos on the exterior of the negative-pressure source 102. Additionally, the negative-pressure source 102 may allow the use of low-cost surface mount pressure transducers. In embodiments where one or more of the front layer 218, the rear layer 220, the front outer layer 700, and the rear outer layer 702 are transparent, the negative-pressure source 102 can be easily inspected for fluid contamination. In embodiments where one or more of the front layer 218, the rear layer 220, the front outer layer 700, and the rear outer layer 702 are vapor permeable, condensation occurring within the negative-pressure source 102 may be evaporated out of the negative-pressure source 102.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 104, the container 106, or both may be eliminated or separated from other components for manufacture or sale. In other example configurations, the controller 112 may also be manufactured, configured, assembled, or sold independently of other components.

## Claims

1. A negative-pressure therapy device comprising:
a formed front layer (218);
a pump (406) having a fluid inlet (434) and a fluid outlet (436);
a pressure transducer (408) fluidly coupled to the ambient environment and the pump (406);
a formed rear layer (220) configured to be coupled to the formed front layer (218) to form an interior space (222) having the pressure transducer (408) and the pump (406) disposed therein;
an exhaust port (206) fluidly coupled to the fluid outlet of the pump (406);
a negative-pressure port (202) fluidly coupled to the fluid inlet (434) of the pump (406); and
an atmospheric pressure measurement port (204);
**characterised in that** the formed front layer (218) and the formed rear layer (220) are made from a flexible polymer film and allow for the negative-pressure therapy device to be conformable.

2. The negative-pressure therapy device of claim 1, wherein the formed front layer (218) and the formed rear layer (220) are formed from polyurethane.

3. The negative-pressure therapy device of claim 1, wherein the exhaust port (206) and the fluid outlet (436) are fluidly coupled to the interior space (222), the exhaust port (206) configured to generate a back pressure to inflate the interior space (222).

4. The negative-pressure therapy device of claim 1, further comprising a manifold (410) fluidly coupled to the pump (406), the pressure transducer (408), and the negative-pressure port (202).

5. The negative-pressure therapy device of claim 1, wherein the pressure transducer (408) is fluidly coupled to the atmospheric pressure measurement port (204) by a fluid path isolated from the interior space (222).

6. The negative-pressure therapy device of claim 1, wherein:
the fluid outlet (436) of the pump (406) is fluidly coupled to the exhaust port (206) by a fluid path that is isolated from the interior space (222);
the fluid inlet (434) of the pump (406) and the negative-pressure port (202) are fluidly coupled to the interior space (222); and
the pressure transducer (408) is fluidly coupled to the atmospheric measurement port (204) by a fluid path that is isolated from the interior space (222).

7. The negative-pressure therapy device of claim 6, wherein the fluid path between the fluid outlet (436) and the pump exhaust port (206) comprises a chamber (1704) fluidly coupled to the fluid outlet (436) on a first end and to the pump exhaust port (206) on a second end.

8. The negative-pressure therapy device of claim 7, further comprising an outer rear layer (702) disposed over the formed rear layer (220), the outer rear layer (702) forming the chamber (1704) between the outer rear layer (702) and the formed rear layer (220).

## Patentansprüche

1. Eine Unterdrucktherapievorrichtung, umfassend:
eine geformte Frontschicht (218);
eine Pumpe (406) mit einem Fluideinlass (434) und einem Fluidauslass (436);
einen Druckwandler (408), der strömungstechnisch an die Umgebung und die Pumpe (406) gekoppelt ist;
eine geformte Rückschicht (220), die konfiguriert ist, um an die geformte Frontschicht (218) gekoppelt zu werden, um einen Innenraum (222) zu bilden, in dem der Druckwandler (408) und die Pumpe (406) angeordnet sind;
einen Abgasanschluss (206), der strömungstechnisch an den Fluidauslass der Pumpe (406) gekoppelt ist;
einen Unterdruckanschluss (202), der strömungstechnisch an den Fluideinlass (434) der Pumpe (406) gekoppelt ist; und
einen Messanschluss für atmosphärischen Druck (204);
**dadurch gekennzeichnet, dass** die geformte Frontschicht (218) und die geformte Rückschicht (220) aus einer flexiblen Polymerfolie hergestellt sind und es ermöglichen, dass die Unterdrucktherapievorrichtung anpassbar ist.

2. Die Unterdrucktherapievorrichtung nach Anspruch 1, wobei die geformte Frontschicht (218) und die geformte Rückschicht (220) aus Polyurethan geformt sind.

3. Die Unterdrucktherapievorrichtung nach Anspruch 1, wobei der Abgasanschluss (206) und der Fluidauslass (436) strömungstechnisch an den Innenraum (222) gekoppelt sind, wobei der Abgasanschluss (206) konfiguriert ist, um einen Rückdruck zu erzeugen, um den Innenraum (222) aufzublasen.

4. Die Unterdrucktherapievorrichtung nach Anspruch 1, ferner umfassend einen Verteiler (410), der strömungstechnisch an die Pumpe (406), den Druckwandler (408) und den Unterdruckanschluss (202) gekoppelt ist.

5. Die Unterdrucktherapievorrichtung nach Anspruch 1, wobei der Druckwandler (408) über einen von dem Innenraum (222) isolierten Fluidpfad strömungstechnisch an den Messanschluss für atmosphärischen Druck (204) gekoppelt ist.

6. Die Unterdrucktherapievorrichtung nach Anspruch 1, wobei:
der Fluidauslass (436) der Pumpe (406) strömungstechnisch über einen von dem Innenraum (222) isolierten Fluidpfad an den Abgasanschluss (206) gekoppelt ist;
der Fluideinlass (434) der Pumpe (406) und der Unterdruckanschluss (202) strömungstechnisch an den Innenraum (222) gekoppelt sind; und
der Druckwandler (408) strömungstechnisch über einen von dem Innenraum (222) isolierten Fluidpfad an den Messanschluss für atmosphärischen Druck (204) gekoppelt ist.

7. Die Unterdrucktherapievorrichtung nach Anspruch 6, wobei der Fluidpfad zwischen dem Fluidauslass (436) und dem Pumpenauslassanschluss (206) eine Kammer (1704) umfasst, die an einem ersten Ende strömungstechnisch an den Fluidauslass (436) gekoppelt ist und an einem zweiten Ende an den Pumpenauslassanschluss (206) gekoppelt ist.

8. Die Unterdrucktherapievorrichtung nach Anspruch 7, ferner umfassend eine äußere Rückschicht (702), die über der geformten Rückschicht (220) angeordnet ist, wobei die äußere Rückschicht (702) die Kammer (1704) zwischen der äußeren Rückschicht (702) und der geformten Rückschicht (220) bildet.

## Revendications

1. Dispositif de thérapie par pression négative, comprenant :
une couche avant formée (218) ;
une pompe (406) ayant une entrée de fluide (434) et une sortie de fluide (436) ;
un transducteur de pression (408) accouplé fluidiquement à l'environnement ambiant et à la pompe (406) ;
une couche arrière formée (220) conçue pour être accouplée à la couche avant formée (218) pour former un espace intérieur (222) ayant le transducteur de pression (408) et la pompe (406) disposés dans celui-ci ;
un orifice d'échappement (206) accouplé fluidiquement à la sortie de fluide de la pompe (406) ;
un orifice de pression négative (202) accouplé fluidiquement à l'entrée de fluide (434) de la pompe (406) ; et
un orifice de mesure de pression atmosphérique (204) ;
**caractérisé en ce que** la couche avant formée (218) et la couche arrière formée (220) sont faites à partir d'un film polymère flexible et permettent au dispositif de thérapie par pression négative d'être conformable.

2. Dispositif de thérapie par pression négative selon la revendication 1, dans lequel la couche avant formée (218) et la couche arrière formée (220) sont formées de polyuréthane.

3. Dispositif de thérapie par pression négative selon la revendication 1, dans lequel l'orifice d'échappement (206) et la sortie de fluide (436) sont accouplés fluidiquement à l'espace intérieur (222), l'orifice d'échappement (206) étant conçu pour générer une contre-pression afin de gonfler l'espace intérieur (222).

4. Dispositif de thérapie par pression négative selon la revendication 1, comprenant en outre un collecteur (410) accouplé fluidiquement à la pompe (406), au transducteur de pression (408) et à l'orifice de pression négative (202).

5. Dispositif de thérapie par pression négative selon la revendication 1, dans lequel le transducteur de pression (408) est accouplé fluidiquement à l'orifice de mesure de pression atmosphérique (204) par un trajet de fluide isolé de l'espace intérieur (222).

6. Dispositif de thérapie par pression négative selon la revendication 1, dans lequel :
la sortie de fluide (436) de la pompe (406) est accouplée fluidiquement à l'orifice d'échappement (206) par un trajet de fluide qui est isolé de l'espace intérieur (222) ;
l'entrée de fluide (434) de la pompe (406) et l'orifice de pression négative (202) sont accouplés fluidiquement à l'espace intérieur (222) ; et
le transducteur de pression (408) est accouplé fluidiquement à l'orifice de mesure atmosphérique (204) par un trajet de fluide qui est isolé de l'espace intérieur (222).

7. Dispositif de thérapie par pression négative selon la revendication 6, dans lequel le trajet de fluide entre la sortie de fluide (436) et l'orifice d'échappement de pompe (206) comprend une chambre (1704) accouplée fluidiquement à la sortie de fluide (436) sur une première extrémité et à l'orifice d'échappement de pompe (206) sur une seconde extrémité.

8. Dispositif de thérapie par pression négative selon la revendication 7, comprenant en outre une couche arrière externe (702) disposée sur la couche arrière formée (220), la couche arrière externe (702) formant la chambre (1704) entre la couche arrière externe (702) et la couche arrière formée (220).
